Europäisches Patentamt

(19) European Patent Office

Office européen des brevets

(11) Publication number: 0 134 318
A2

(12) # EUROPEAN PATENT APPLICATION

(21) Application number: 83303606.4

(22) Date of filing: 22.06.83

(51) Int. Cl.⁴: **A 61 K 9/22**
**A 61 K 37/26**

(30) Priority: 10.06.83 CA 430195

(43) Date of publication of application:
20.03.85 Bulletin 85/12

(84) Designated Contracting States:
AT BE CH DE FR GB IT LI LU NL SE

(71) Applicant: CONNAUGHT LABORATORIES LIMITED
1755 Steeles Avenue West
Willowdale Ontario M2N 5T8(CA)

(72) Inventor: Goosen, Mattheus Florentius Albertus
18 Laxton Avenue
Toronto Ontario, M6K 1K9(CA)

(72) Inventor: Sun, Anthony Mein-Fang
4 Barkwood Crescent
Willowdale Ontario, M2H 3G6(CA)

(74) Representative: Burford, Anthony Frederick et al,
W.H. Beck, Greener & Co. 7 Stone Buildings Lincoln's Inn
London WC2A 3SZ(GB)

(54) Controlled release of injectable and implantable insulin compositions.

(57) Long lasting injectable and implantable insulin composi-
tions are provided in the form of an insulin-biodegradable
polymer matrix. The biodegradable polymer is preferably a
lactic acid polymer and may be provided in the form of
microbeads or pellets.

Croydon Printing Company Ltd.

## CONTROLLED RELEASE OF INJECTABLE AND
## IMPLANTABLE INSULIN COMPOSITIONS

The present invention relates to long-lasting injectable and implantable insulin compositions.

Normoglycemia in diabetic patients is controlled by injection of insulin into the body of the patient. Insulin injections several times a day are required, although more recently long-lasting Ultralente and Protamine zinc insulin have decreased the frequency of injection for some diabetics to once every day. These insulin preparations contain protamine and/or zinc which bind insulin strongly thus delaying its absorption by the body.

There is a need for a system which would permit the administration of one large dose of insulin at widely-spaced intervals of weeks or even months. Such a large dose would need to be associated with some means of permitting the administered insulin to be released into the blood stream at a controlled rate over a period of time, so as to be effective over that period.

It has now been found that there can be produced a biodegradable composition comprising insulin and a biodegradable polymer matrix which is able to achieve long-lasting release of insulin into the blood stream sufficient to control blood sugar levels upon injection or implantation.

The composition may be provided in a variety of physical forms, including microbeads and pellets. The insulin release rate and duration of action of the microbeads or pellets are controlled by: i) the insulin loading (weight insulin/weight polymer), ii) the available surface area for insulin release, iii) the geometry or shape of the matrix, and iv) the insulin-polymer dose. For example, in the case of a pellet, the available surface area for insulin release may be controlled by partially coating the pellet with an impermeable film of a biocompatible polymer. The insulin entrapped within the matrix is able to be released over a period of several days to several weeks, depending on the design parameters and achieve blood sugar level control over that period.

In the present invention, a biodegradable matrix of a biodegradable polymer and insulin is provided. The biodegradable polymer is any convenient biodegradable polymer which is soluble in organic solvents to permit the formation of the matrix therewith. Suitable biodegradable polymers include polylactic acid and copolymers of lactic acid and glycolic acid having a lactic acid content of from about 10% to about 90 wt.%.

The invention is described hereinafter mainly with reference to polylactic acid but it will be understood that the principles described herein apply to any biodegradable polymer from which the matrix may be formed.

The polylactic acid which may be used in this invention may have a wide range of molecular weight, generally in the range of about $3 \times 10^4$ to about $1 \times 10^6$, preferably about $5 \times 10^4$ to about $1 \times 10^5$.

Polylactic acid and insulin are processed under relatively mild conditions to form the matrix of polylactic acid containing entrapped insulin crystals. By mixing normally impermeable polymers, such as polylactic acid, with powdered macromolecules, such as insulin crystals, in volatile solvents, a series of interconnecting channels, large enough to permit macromolecular release, is formed within the polymer matrix.

Injectable insulin-polymer microbeads may be prepared by emulsifying an organic suspension containing polylactic acid, methylene chloride or other suitable water-immiscible organic solvent and insulin crystals in a rapidly stirring, continuous aqueous phase and then reducing the pressure to evaporate the organic solvent. The insulin-polymer microbeads are then recovered by filtration and dried. The microbeads so formed usually have a diameter of about 10 to about 300 $\mu$m.

Implantable insulin-polymer pellets may be prepared by pouring an organic suspension containing polylactic acid, methylene chloride or other suitable water-immiscible organic solvent and insulin crystals, into glass molds. After drying, the pellets are removed from the molds, trimmed and, if necessary, further filled with insulin-polymer solution to give the desired pellet geometry. The pellets so formed usually are cylindrical and have a length from about 2 to about 15 mm and a thickness of about 1 to about 5 mm.

The insulin release rate and duration of action of the preparations may be controlled by the insulin loading, the available surface area for insulin release, the geometry or shape, and the dose amount of insulin-polymer used. While the insulin-polylactic acid matrix is a biodegradable one, insulin release occurs essentially by a solution-diffusion mechanism, as with non-erodible systems. The polylactic acid matrix degrades in 1 to 4 months, after all of the bioactive agent has been released.

A higher insulin loading (weight insulin/weight polymer) while increasing the insulin release rate actually decreases the duration of action since it increases the porosity of the polymer matrix (due to lower weight percent polymer), making it easier for aqueous solution to diffuse into the matrix to dissolve the hormone and for the dissolved hormone to diffuse back out. The reduced travelling time means that all of the insulin is released from the matrix in a short period of time. A reduced insulin loading would result in a less porous matrix, a lower insulin release rate, and thus a longer duration of action. Generally, the quantity of insulin used is about 5 to about 60 wt.% of the insulin-polylactic acid matrix. The release rate of insulin from the matrix usually is about 1 to about 50 IU/day.

In the case of an insulin-polymer pellet, the insulin release rate may also be decreased by decreasing the available surface area for release. This may be

4

0134318

accomplished, for example, by coating one side of a flat cylindrical insulin-polylactic acid pellet with an impermeable polylactic acid film. This has the additional advantage of increasing the duration of action of the pellet since insulin solution-diffusion would occur only from the uncoated side of the pellet. As more of the insulin was released, the dissolution-diffusion front would move gradually inward leaving behind a porous polymer matrix consisting of interconnecting channels. The increase in the diffusion path would result in a longer travelling time and, therefore, a decrease in the release rate with time. One approach which can be used to approximate a constant insulin release rate (zero-order kinetics) would be to compensate for the increasing diffusional distance by choosing a pellet geometry that would increase the available surface area for drug release as a function of time. With non-erodible systems, the best results have been from a cylinder sector that releases drug only from the inside surface or a hemisphere that is laminated with an impermeable coating in all places except for a small cavity in the centre face.

In addition to the product of the invention being biocompatible, inexpensive and versatile, the matrix material has a long shelf-life. Insulin-polylactic acid microbeads or pellets, for example, may be stored dry at 4°C for many months prior to injection or implantation without loss of effectiveness.

Lactic/glycolic acid copolymers, with the lactic acid content varying from 10% to 90% may also be used in place of polylactic acid. The copolymer biodegrades faster due to the presence of glycolic acid.

The invention is illustrated by the following Examples:

Example 1

This Example illustrates the preparation of injectable insulin-polymer microbeads.

TABLE 1

| Example | Catalyst | Temp, $^{o}$C | WHSV | Conversion wt. % | Approximate WHSV to achieve 100 % conv. | Approximate activity enhancement factor |
|---------|----------|---------------|------|------------------|------------------------------------------|------------------------------------------|
| 1 | HZSM-5 | 300 | 1.2 | 46 | 0.6 | -- |
| 2 | HZSM-5 | 300 | 1.5 | 35 | 0.5 | -- |
| 3 | HZSM-5 | 300 | 6.2 | 7 | 0.5 | -- |
| 4 | BF$_3$ HZSM-5 | 300 | 9.0 | 100 | 9.0 | 15-20 |
| 5 | BF$_3$ HZSM-5 | 280 | 2.3 | 100 | 2.3 | 4-5 |

hood at 20°C. After 15 min. the upper sides of the partially dried pellet were gently pressed in and then the pellet was allowed to dry for another 3 hours. The dried pellet was removed from the mold and the air dried side was further filled with viscous insulin-PLA solution (50 mg insulin in 0.2 ml of 20% PLA) to give a flat cylindrical pellet. The pellet, after drying at 20°C for 1 hour and under vacuum for another hour, was weighed and trimmed to give an insulin-PLA pellet 11 mm diam. x 2 mm high, weighing 100 mg and containing 55% w/w insulin (1400 units).

 b.  Hemispherical Pellets

The same procedure as in Example 2(a) is followed except that hemispherical glass molds were used. The resultant insulin-PLA hemispheres, had a radius of 4 mm, weighed 100 mg and contained 55% w/w insulin.

Example 4

This Example illustrates the effect on blood sugar levels obtained by injection of insulin-polymer microbeads.

Insulin-polymer microbeads produced as described in Example 1 (10 to 100 $\mu$m diam.) were mixed with 1 ml of 50% glycerol in saline and then injected into the lower flanks of diabetic rats using an 18-gauge needle fitted into a syringe. The blood sugar levels were tested at different dosage levels over a period of 17 days. The results obtained are reproduced in the following Table I:

TABLE I

| Microbead dose mg | Day | 0 | 1 | 3 | 5 | 7 | 9 | 11 | 14 | 17 | (No. of Tests) |
|---|---|---|---|---|---|---|---|---|---|---|---|
| | | | | | Blood Glucose* (mg/dL) | | | | | | |
| 25 | | 372 | 74 | 113 | 229 | 361 | 384 | 436 | 325 | 273 | (N=3) |
| 50 | | 380 | 44 | – | 101 | 96 | 229 | 457 | 414 | 498 | (N=4) |
| 100 | | 320 | 32 | – | 94 | 82 | 91 | 151 | 338 | 351 | (N=3) |

* Fasting blood glucose

As can be seen from the results of Table I, a single injection of insulin-polymer microbeads lowered the animals blood glucose for about 2 weeks. In addition, the higher the dose, the longer the duration of action.

Example 5

This Example illustrates the effect on blood sugar levels obtained by administration of insulin-polymer pellets.

Insulin-polymer pellets, produced by the procedure in Example 2, were cut into quarter sections each weighing 30 mg and containing about 250 units of insulin. Each quarter section was implanted subcutaneously in the flanks of chemically-induced diabetic rats, by means of a small incision in the skin. The non-fasted blood glucose levels were monitored over a 14-day period and compared to those for untreated rats. The results are reproduced in the following Table II:

TABLE II

| Days | 0 | 1 | 3 | 7 | 9 | 11 | 12 | 14 |
|------|---|---|---|---|---|----|----|----|
| Blood sugar levels (mg%) | | | | | | | | |
| - treated diabetic rats (N=6) | 288 | 106 | 42 | 43 | 44 | 97 | 93 | 360 |
| - control diabetic rats (N=4) | 380 | 385 | 390 | 387 | 390 | 392 | 385 | 400 |

As may be seen from the results of the above Table II, after implantation of the pellets, the blood glucose levels fell from 288 mg/dL to 106 mg/dL within 24 hours and remained stable for almost 14 days. The control animals did not display any decrease in their high blood glucose levels.

Example 6

This Example illustrates the effect on body weights obtained by injection of insulin-polymer microbeads.

Diabetic rats were injected with insulin-polymer microbeads produced as described in Example 1 and the

As can be seen from the above table, the catalyst of Example 7 had a substantially lower alpha activity than the catalyst of Example 6, yet it had enhanced activity for the conversion of methanol. The same is true of regard to the catalyst of Example 10 as compared to the catalyst of Example 9. Please note that the treatment with ammonium fluoride, i.e. Example 8, and hydrogen fluoride, i.e. Example 12 did not result in enhancement of methanol conversion activity and, in fact, Example 12 shows an actual decline relative to the untreated material.

Example 11 depicts the results of incorporating an HZSM-5 into an alumina binder prior to treatment with boron trifluoride and, as can be seen, a catalyst was obtained with an alpha of 6 and only slightly enhanced activity at 300°C. The $BF_3$ appears to react much more rapidly with the alumina binder than with the ZSM-5.

EXAMPLES 13-16

These examples will illustrate the criticality of the silica-to-alumina ratio of the ZSM-5 type zeolite. In Examples 13 and 14, a crystalline ZSM-5 zeolite having a silica-to-alumina ratio of 800:1 was employed. In Examples 15 and 16, a similar material but having a silica-to-alumina ratio of 1600 was employed. The method of activation with boron trifluoride was the same as previously described. The results are shown in the following table.

the insulin release rate and duration of action of pellets implanted in diabetic rats.

Insulin-polymer pellets produced by the procedures of Examples 3(a) and (b) and containing about 1,400 units of insulin were partially coated with an impermeable film by being dipped into 20% w/v polylactic acid. The available surface area for insulin release ranged from 450 mm² to 2 mm² as shown in Table V, below. An uncoated pellet with 450 mm² available surface area for insulin release, had a very high insulin release rate (up to 1,000 µU insulin/ml blood) and a very short duration of action ($<$ 1 week). Animals with these implants died from insulin shock within 5 days. On the other hand, pellets with the same insulin content and only 13 mm² of available surface area released a negligible amount of insulin (less than 20 µU/ml found in blood) and were ineffective in lowering the animals blood glucose levels. The results show that with the particular insulin loading, (50 mg insulin/40 mg polymer) used in Example 2, the optimum available surface area for insulin release is between 100 and 200 mm².

TABLE V

| Pellet Geometry | Available Surface Area for Insulin Release mm² | Duration of Action Wks | In Vivo Insulin Release Rate + (Negligible) $\rightarrow$ +++++ (Very High) |
|---|---|---|---|
| Flat Cylinder | 450 | $<$1 | +++++ |
|  | 225 | 1 | ++++ |
|  | 150 | 2 | +++ |
|  | 13 | Ineffective | + |
| Hemisphere | 7 | Ineffective | + |
|  | 2 | Ineffective | + |

In summary of this disclosure, the present invention provides an insulin-polymer matrix which is effective for long term control of blood sugar levels.

10

**0134318**

Modifications are possible within the scope of this invention as defined in the following Claims.

CLAIMS

1. A biodegradable composition suitable for administration to a body, characterized by insulin and a biodegradable polymer matrix.

2. The composition claimed in claim 1, characterized in that the polymer is a lactic acid polymer.

3. The composition claimed in claim 2, characterized in that the lactic acid polymer has a molecular weight of $3 \times 10^4$ to $1 \times 10^6$.

4. The composition claimed in claim 3, characterized in that the lactic acid polymer has a molecular weight of $5 \times 10^4$ to $1 \times 10^5$.

5. The composition claimed in any one of claims 2 to 4, characterized in that the lactic acid polymer is polylactic acid.

6. The composition claimed in any one of claims 2 to 5, characterized in that the lactic acid polymer is a copolymer of lactic acid and glycolic acid containing 10 to 90wt.% lactic acid.

7. The composition claimed in any one of claims 1 to 6, characterized in that the insulin comprises 5 to 60% w/w of the composition.

8. The composition claimed in any one of claims 1 to 7, characterized by the physical form of microbeads of diameter 10 to 300 μm.

9. The composition claimed in any one of claims 1 to 7, characterized by the physical form of pellets.

10. The composition claimed in claim 9, characterized in that the pellets are cylindrical, have a length of 2 to 15 mm and have a thickness of 1 to 5 mm.

11. A process for the preparation of a biodegradable composition useful for the control of blood sugar levels in a body, characterized by mixing powdered insulin with a solution of a biodegradable polymer in a volatile organic solvent for the polymer, and removing the solvent to provide a polymer matrix containing the insulin.

12

0134318

12.      The process claimed in claim 11, characterized by emulsifying an organic suspension of the polymer, volatile organic solvent and insulin in a continuous aqueous phase, evaporating the organic solvent to form a suspension of microbeads in the aqueous phase, and filtering the microbeads from the aqueous phase.

13.      The process claimed in claim 11, characterized by pouring an organic suspension of the polymer, volatile organic solvent and insulin into a mold, evaporating the organic solvent to form a solid pellet in the mold, and removing the pellet from the mold.

## CLAIMS

1.      A method of forming a biodegradable composition suitable for administration to a body, characterized by distributing insulin throughout a biodegradable polymer.

2.      The method claimed in claim 1, characterized in that the polymer is a lactic acid polymer.

3.      The method claimed in claim 2, characterized in that the lactic acid polymer has a molecular weight of $3 \times 10^4$ to $1 \times 10^6$.

4.      The method claimed in claim 3, characterized in that the lactic acid polymer has a molecular weight of $5 \times 10^4$ to $1 \times 10^5$.

5.      The method claimed in any one of claims 2 to 4, characterized in that the lactic acid polymer is polylactic acid.

6.      The method claimed in any one of claims 2 to 5, characterized in that the lactic acid polymer is a copolymer of lactic acid and glycolic acid containing 10 to 90wt.% lactic acid.

7.      The method claimed in any one of claims 1 to 6, characterized in that the insulin comprises 5 to 60% w/w of the composition.

8.      The method claimed in any one of claims 1 to 7, characterized in that the insulin is mixed in powdered form with a solution of the biodegradable polymer in a volatile organic solvent for the polymer and the solvent is removed.

9.      The method claimed in claim 8, characterized in that an organic suspension of the polymer, solvent and insulin is emulsified in a continuous aqueous phase and removal of the solvent by evaporation forms a suspension of microbeads in the aqueous phase.

10.      The method claimed in claim 9, characterized in that the microbeads have a diameter of 10 to 300 $\mu$m.

11.      The method claimed in claim 8, characterized in that an organic suspension of the polymer, solvent and insulin is poured into a mold, removal of the solvent by evaporation forms a solid pellet in the mold, and the pellet is removed from the mold.

12.      The method claimed in claim 11, characterized in
that the pellets are cylindrical, have a length of 2 to
15 mm and a thickness of 1 to 5 mm.